# EUROPÄISCHE PATENTSCHRIFT

(11) **EP 2 491 107 B1**
(45) Veröffentlichungstag und Bekanntmachung des Hinweises auf die Patenterteilung: **23.01.2013**
(21) Anmeldenummer: 10771704.3
(22) Anmeldetag: 19.10.2010
(51) Int. Cl.: C12M 1/00

(54) **SCHLAUCH-PHOTOBIOREAKTOR**
TUBULAR PHOTOBIOREACTOR
PHOTO-BIORÉACTEUR TUBULAIRE

(30) Priorität: 20.10.2009 DE 102009045851
(43) Veröffentlichungstag der Anmeldung: 29.08.2012
(73) Patentinhaber: Wacker Chemie AG, 81737 München (DE)
(72) Erfinder: MÜLLER-REES, Christoph, 82049 Pullach (DE); PFALLER, Rupert, 80639 München (DE); WALTER, Christian, 84489 Burghausen (DE); COTTA, Fritz, 06217 Merseburg (DE)
(74) Vertreter: Schuderer, Michael
(86) Internationale Anmeldenummer: PCT/EP2010/065739
(87) Internationale Veröffentlichungsnummer: WO 2011/048108

(56) Entgegenhaltungen:
- WO-A-99/28018
- WO-A-2007/129327
- WO-A1-2008/145719
- DE-U1- 29 707 043
- US-A- 5 958 761

## Beschreibung

Die Erfindung betrifft einen Schlauch-Photobioreaktor sowie die Kultivierung von phototrophen Makro- oder Mikroorganismen unter Verwendung eines solchen Photobioreaktors.

Photobioreaktoren werden zur großtechnischen Produktion von phototrophen Organismen, z.B. Cyanobakterien oder Mikroalgen, beispielsweise Spirulina, Chlorella, Clamydomonas oder Haematococcus eingesetzt. Derartige Mikroalgen sind in der Lage, Lichtenergie, CO₂ und Wasser in Biomasse umzuwandeln. Photobioreaktoren der ersten Generation nutzen das Sonnenlicht als Lichtquelle. Die Reaktoren bestehen aus großen offenen Beckenanlagen in vielerlei Formgestalt, beispielsweise Rundbeckenanlagen mit Durchmessern bis zu 45 m und umlaufenden Mischarmen. Diese Reaktoren sind im Allgemeinen aus Beton oder Kunststoffen gefertigt. Geschlossene Bioreaktoren werden ebenfalls in vielerlei Formen eingesetzt. Bei den geschlossenen Bioreaktoren kann es sich um Plattenbioreaktoren, Rohrbioreaktoren, (Blasen)Säulenbioreaktoren oder Schlauchbioreaktoren handeln. Dieser Reaktortyp wird aus transparenten oder transluzenten Materialien gefertigt, wie Glas oder Kunststoff.

Geschlossene Photobioreaktoren bieten den Vorteil, dass die Herstellung der Biomasse unter kontrollierten Bedingungen erfolgen kann und die Kontamination der Kultur zurückgedrängt werden kann. Zur Verbesserung des Lichteintrags in die Kulturen, mittels Erhöhung des Oberflächen/Volumen-Verhältnisses, werden Rohr- bzw. Schlauchphotobioreaktoren empfohlen, wobei zur Verminderung des Platzbedarfes die Rohre bzw. Schläuche um ein zylinderförmiges Gerüst helikal gewickelt werden.

In der WO 2007/129327 A1 wird ein Photobioreaktor beschrieben, welcher mindestens zwei transparente, spiralförmige Röhren enthält, welche jeweils um ein zylindrisches Trägergerüst gewickelt sind, wobei die einzelnen Röhrenelemente über deren freie Enden miteinander verbunden werden. Die Röhren werden vorzugsweise aus Siliconen gefertigt. Die Beleuchtung des Reaktors erfolgt mittels röhrenförmiger Beleuchtungselemente, welche in den ringförmigen Lücken zwischen den einzelnen spiralförmigen Röhrenelementen angeordnet sind. Die Temperierung des Röhrensystems erfolgt mittels eines außerhalb der helikalen Anordnung angeordneten Wärmetauschers. Problematisch ist hier die inhomogene Temperaturführung aufgrund des außerhalb angeordneten Wärmetauschers und der bei zylindrisch-helikaler Geometrie unterschiedlichen Strahlungsintensität im oberen und unteren Teil des Reaktors. In der EP 239272 B1 wird ein Photobioreaktor mit einer aufrecht stehenden Kernstruktur beschrieben, welche zylindrisch oder in Form eines Konus vorliegen kann. Um die Außenseite der Kernstruktur ist ein transparentes Rohr helikal gewickelt. Als Rohrmaterial wird Polyethylen empfohlen. Zur Temperierung wird ein außerhalb der helikalen Konstruktion angeordneter Wärmetauscher empfohlen. Die Beleuchtung erfolgt an der Außenseite mittels Sonneneinstrahlung. Zur Steigerung der Lichtintensität wird empfohlen, die Innenseite der Kernstruktur mit einer reflektierenden Beschichtung zu versehen bzw. künstliche Lichtquellen in die Kernstruktur einzubauen. Nachteilig bei dieser Ausführungsform ist die bei zylindrischer Kernstruktur unterschiedliche Strahlungsintensität, und die unzureichende Temperierung mit externem Wärmetauscher.

Die GB 2205581 A beschreibt einen Photobioreaktor mit zylindrischer Kernstruktur oder einer Kernstruktur in Form eines Kegelstumpfes. Um diese Kernstruktur sind außen ein oder zwei transparente Kunststoffschläuche helikal gewickelt. Alternativ dazu kann auch jeweils ein Schlauch an der Innenseite bzw. Außenseite der Kernstruktur helikal angeordnet sein. Als Schlauchmaterial wird transparenter Kunststoff oder Glas empfohlen. Die Beleuchtung erfolgt mit Sonnenlicht bzw. künstlichen Lichtquellen, welche zwischen Innenseite und Außenseite der Kernstruktur angebracht sind. Es werden keine Einrichtungen zur Temperierung des Kultivierungsmediums beschrieben. In der WO 2008/097845 A1 wird ein Photobioreaktor mit zylindrischer Kernstruktur beschrieben, wobei um die Kernstruktur helikal gewundene Schläuche aus transparentem Kunststoff angeordnet sind. Die Temperierung erfolgt über einen an der Innenseite der Kernstruktur angebrachten Wärmeaustauscher.

Die DE 29707043 U1 beschreibt einen Photobioreaktor mit einer an einem Trägergestell umlaufenden, transparenten, rohrförmigen Leitung, welche mit Kulturmedium befüllt ist. Zur Steigerung der Lichtversorgung ist innerhalb des Trägergestells eine Lichtquelle angeordnet und am oberen Ende des Trägergestells eine Sammellinse. Zur Kühlung des umlaufenden Kulturmediums wird empfohlen, zur Klimatisierung des Innenraumes, im Sockel des Trägergestells Luftschlitze anzubringen. In der US 5958761 wird ein zylindrischer Bioreaktor zur Algenkultivierung beschrieben, welcher aus Glas gefertigt ist und einen äußeren Zylinder mit größerem Durchmesser umfasst, und einen inneren Glaszylinder mit kleinerem Durchmesser. Der innere Zylinder wird mit der Algenkultur befüllt und mit einer Mischvorrichtung ausgerüstet. Zur Verbesserung der Lichteinstrahlung ist der äußere Zylinder mit einer Flüssigkeit befüllt, deren Brechungsindex dem geometrischen Verhältnis von Innen- und Außenzylinder angepasst ist. Diese Flüssigkeit kann auch als Kühlmedium dienen. Zur weiteren Verbesserung der Lichteinstrahlung wird empfohlen, den Glaszylinder in eine verspiegelte Parabolrinne einzulegen. Nachteilig sind die ungünstigen Strömungsverhältnisse in dem Innenzylinder, welche den Einbau einer konstruktiv aufwändigen Rühreinheit bedingen.

Vor diesem Hintergrund bestand die Aufgabe, einen Photobioreaktor zur Verfügung zu stellen, welcher sich gegenüber dem vorgenannten Stand der Technik dadurch auszeichnet, dass die Lichteinstrahlung und Temperierung über das gesamte Reaktorvolumen möglichst gleichmäßig erfolgt.

WO 99/28018 offenbart einen Schlauch-Photobioreaktor mit einem Kegelstumpf-förmigen Trägergestell (46), auf dessen Außenseite ein transparentes Rohr helikal aufgewickelt ist. Eine als Haube ausgebildete Linse unterbindet zu große Temperaturschwankungen im Photobioreaktor.

US 5958761 offenbart einen Photobioreaktor, der aus einem zylindrischen Behältnis mit einem innenliegenden, koaxial verlaufenden zylindrischen Körper besteht. Der innenliegende Zylinder ist mit Kultivierungsmittel gefüllt, der außenliegende Zylinder ist mit einem Wärmetauschmedium durchströmt, um die Temperatur des Kultivierungsmediums zu kontrollieren und regulieren.

Gegenstand der Erfindung ist ein Schlauch-Photobioreaktor mit einer Kegelstumpf-förmigen Kernstruktur und einem oder mehreren transparenten oder transluzenten Schläuchen, welche um die Außenseite und/oder Innenseite der Kernstruktur helikal aufgewickelt sind, dadurch gekennzeichnet, dass der transparente oder transluzente Schlauch mindestens zwei Kammern aufweist, von denen mindestens eine von dem Kultivierungsmedium durchströmt wird und mindestens eine von einem Wärmetauschmedium durchströmt wird, und die Wickelungen des Schlauches in Abstand Zveinander gelegt werden, und die Förderung des Kutivierungsmediums gepulst er follgt.

Der Schlauch-Photobioreaktor eignet sich zur Kultivierung von phototrophen Makro- oder Mikroorganismen in wässrigem Medium. Als phototrophe Organismen werden dabei solche bezeichnet, welche Licht und Kohlendioxid, oder gegebenenfalls noch eine weitere Kohlenstoffquelle, zum Wachstum benötigen. Beispiele für phototrophe Makroorganismen sind Makroalgen, Pflanzen, Moose, Pflanzenzellkulturen. Beispiele für phototrophe Mikroorganismen sind phototrophe Bakterien wie Purpurbakterien und phototrophe Mikroalgen einschließlich Cyanobakterien. Bevorzugt dient der Schlauch-Photobioreaktor der Kultivierung von phototrophen Mikroorganismen, besonders bevorzugt der Kultivierung phototropher Mikroalgen. Geeignete Kultivierungsmedien enthalten neben Wasser und Makro- oder Mikroorganismen vorzugweise noch Nährsalze und/oder Wachstums- oder Produktbildungsfördernde Stoffe, gegebenenfalls organische oder anorganische Kohlenstoffquellen wie beispielsweise Bicarbonate oder Natriumhydrogencarbonat. Das Kultivierungsmedium kann bezüglich des pH-Wertes gegebenenfalls zusätzlich abgepuffert werden.

Als Wärmetauschmedium wird vorzugsweise Wasser eingesetzt.

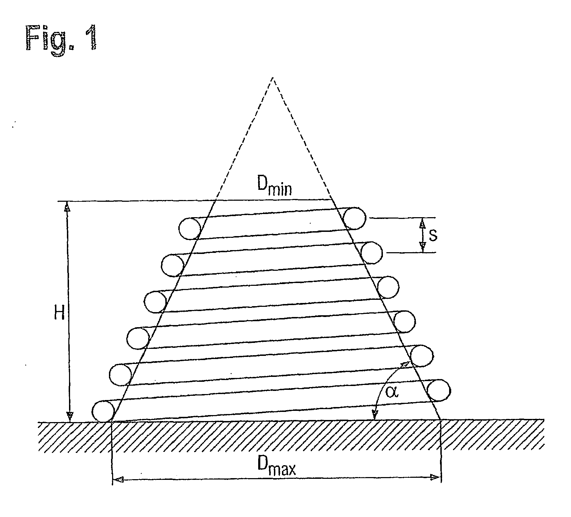

In Figur 1 wird der prinzipielle Aufbau des Schlauch-Photobioreaktors aufgezeigt. Die Kernstruktur hat die Form eines Kegelstumpfes mit einem Durchmesser Dmax am Boden, einem Durchmesser Dmin am oberen Ende in der Höhe H, sowie einem Anstellwinkel α. Unter kegelstumpf-förmiger Kernstruktur sollen dreidimensionale Strukturen verstanden werden mit runder oder polygonaler Grundfläche und im Anstellwinkel α nach innen geneigten Seiten. Bei runder Grundfläche kann diese kreisrund oder oval sein. Polygonale Grundflächen umfassen beliebige Vielecke wie Viereck oder Oktagon. Die Kernstruktur kann auch dahingehend von einem idealen Kegelstumpf abweichen, dass die Seiten nach oben zunehmend nach innen geneigt sind und eine "Iglu-artige" Kernstruktur oder ein "abgeknickter Kegelstumpf" resultiert. Alle diese genannten Ausführungsformen sollen von der Bezeichnung "Kegelstumpf-förmige Kernstruktur" mitumfasst werden.

Die Kernstruktur kann eine durchgehende Oberfläche aufweisen und beispielsweise aus Platten aufgebaut sein. Beispiele hierfür wären (Leicht)Metallplatten wie eine Stahl- oder Aluminiumplatte, oder auch Kunststoffplatten, vorzugsweise transparente Kunststoffplatten, beispielsweise Polyvinylchlorid- oder Polycarbonat-Platten, oder Holzplatten.

Die Kernstruktur kann auch eine durchbrochene Oberfläche aufweisen. Für die Haltekonstruktion können beliebige Materialien wie Holz, Kunststoffe oder Metall eingesetzt werden. Beispielsweise kann sie aus einem Metall- oder Kunststoffgitter aufgebaut sein. Vorzugsweise wird die Kernstruktur aus Metallstützen, gegebenenfalls mit entsprechenden Querverstrebungen aus Metall, aufgebaut. Bevorzugt werden als Metall Stahl oder Leichtmetalle wie Aluminium.

Für die Dimensionierung der Kegelstumpf-förmigen Kernstruktur sind mehrere Faktoren zu berücksichtigen. Der Anstellwinkel α ist im Hinblick auf die optimale Lichternte hin auszulegen. Der Winkel kann fest gewählt werden und im Hinblick auf Aufstellungsort und Gesamtjahres-Lichtausbeute hin optimiert werden. Die Kegelstumpf-förmige Kernstruktur kann auch so konstruiert werden, dass der Anstellwinkel α variiert werden kann, beispielsweise mittels Teleskopstangen als Metallstützen. Bei abgeknicktem Kegelstumpf kann der Anstellwinkel α im oberen Bereich des Kegelstumpfes kleiner gewählt werden als an der Basis. Je größer der Anstellwinkel α, desto geringer ist der Grundflächenbedarf für den Schlauch-Photobioreaktor. Mit zunehmendem Anstellwinkel α nimmt allerdings auch die Verschattung zu. In mitteleuropäischen Breiten ist der optimale jahresintegrale Anstellwinkel für die Nutzung von Solarenergie, gemäß Daten für Solarpanels, bei etwa 30° bis 50°. Der Anstellwinkel α beträgt daher im Allgemeinen von 20° bis < 90°, vorzugsweise 20° bis 70°, besonders bevorzugt 30° bis 50°.

Dmax. und Dmin können im Prinzip beliebig gewählt werden. Für die industrielle Nutzung beträgt der Durchmesser Dmax im Allgemeinen 0,9 m bis 5 m, vorzugsweise 2 m bis 3,5 m. Das Verhältnis von Dmax/Dmin beträgt bei industrieller Nutzung im Allgemeinen von 2 bis 5, vorzugsweise von 2,5 bis 4. Die Höhe der Kegelstumpf-förmigen Kernstruktur beträgt im Allgemeinen 0,5 m bis 5 m, vorzugsweise 0,5 m bis 3 m.

Der transparente oder transluzente Schlauch wird helikal aufgewickelt. Vorzugsweise wird der Schlauch nur an der Außenseite der Kegelstumpf-förmigen Kernstruktur aufgewickelt. Der Schlauch wird vorzugsweise mit einem ansteigenden Winkel um die Kernstruktur gewickelt, wobei der Steigungswinkel von der Dimensionierung der Kernstruktur abhängt. Die Schlauchwicklung kann nur aus einem einzigen durchgehenden Schlauch bestehen. Es können auch mehrere Schlauchmodule zu einem durchgehenden Schlauch zusammengesteckt werden. Die Schlauchwicklung kann auch aus mehreren, nicht miteinander verbundenen Schläuchen zusammengesetzt sein. Eine Schlauchwicklung, welche aus mehreren Schläuchen zusammengesetzt ist, birgt den Vorteil, dass die Abführung des bei der Kultivierung gebildeten Sauerstoffs erleichtert wird.

Der Abstand S, das heißt der Abstand zwischen den Mittelpunkten des Querschnitts zweier übereinanderliegender Schlauchabschnitte, als Maß für den Abstand zwischen den Wicklungen beträgt ≥ 2r, wobei r der Radius des Schlauchquerschnitts ist. Zur Verbesserung der Lichteinstrahlung in die Schläuche können die Wicklungen auch im Abstand gelegt werden. Vorzugsweise beträgt der Abstand S daher 2r ≤ S ≤ 4 r.

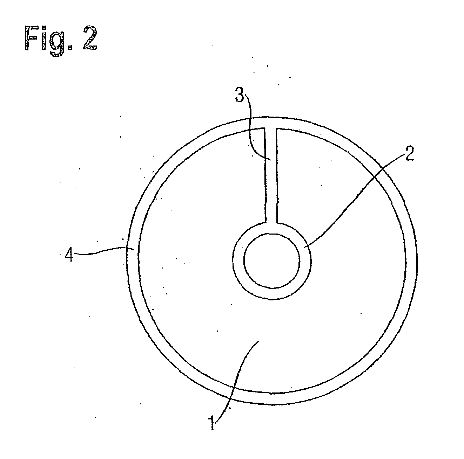

Der transparente Schlauch umfasst zwei oder mehr Kammern, von denen mindestens eine von dem Kultivierungsmedium durchströmt wird und mindestens eine von einem Wärmetauschmedium durchströmt wird. Der Schlauch kann einen polygonalen oder runden Querschnitt aufweisen. Vorzugsweise hat der Schlauch einen kreisförmigen oder ovalen Querschnitt. Der Schlauch kann beispielsweise mittels Einbau von Stegen in zwei oder mehr Kammern unterteilt werden. Beispielsweise können Schläuche eingesetzt werden, welche mittels eines radial verlaufenden Steges in zwei Kammern aufgeteilt werden. Es können auch Schläuche eingesetzt werden, welche einen oder mehrere Innenschläuche enthalten, welche gegebenenfalls jeweils über einen Steg mit dem Außenschlauch verbunden sein können. Es kann aber auch so vorgegangen werden, dass in einen äußeren Schlauch mit größerem Durchmesser ein oder mehrere Schläuche mit geringerem Durchmesser eingelegt werden. Bevorzugt wird ein Schlauch, der aus einem äußeren Schlauch und einem koaxial verlaufenden inneren Schlauch zusammengesetzt ist. Besonders bevorzugt wird ein Schlauch 1 (Doppelschlauch), welcher einen koaxial verlaufenden Innenschlauch 2 enthält, der über einen Steg 3 mit dem äußeren Schlauch 4 verbunden ist; wie der in Fig. 2 abgebildete Doppelschlauch, wobei die Dimensionen und Abmessungen nur prinzipiell dargestellt sind.

Die Dimensionierung der Schläuche hängt von der Dimensionierung der Kegelstumpf-förmigen Kernstruktur ab. Je größer die Kernstruktur dimensioniert ist, umso länger der Schlauch. Der Durchmesser des Schlauches hängt ebenfalls von der Dimensionierung der Kegelstumpf-förmigen Kernstruktur ab. Je geringer die Durchmesser der Kernstruktur sind, umso kleiner sind die Biegeradien des Schlauches. Der Durchmesser des Schlauches ist folglich so zu dimensionieren, dass er um die Kegelstumpf-förmige Kernstruktur helikal gewickelt werden kann, ohne dass der Schlauch abknickt.

Die Länge des Schlauches kann bis zu mehrere hundert Meter, vorzugsweise 50 m bis 100 m, betragen. Bei Längen der Schlauchwicklung ab 100 m wird vorzugsweise so vorgegangen, dass die Schlauchwicklung aus mehreren Schläuchen zusammengesetzt wird, deren Länge vorzugsweise jeweils von 50 bis 100 m beträgt. Die Wandstärken des Schlauches bzw. der Stege, welche den Schlauchinnenraum in getrennte Kammern teilen, und auch die Wandstärke eventueller Innenschläuche hängt von der Dimensionierung des Schlauches ab. Im Allgemeinen betragen die Wandstärken 1 bis 10 mm, vorzugsweise 2 bis 5 mm. Der Durchmesser des Schlauches beträgt im Allgemeinen nicht mehr als 200 mm, vorzugsweise 5 bis 100 mm. Bei den Ausführungsformen mit einem oder mehreren Innenschläuchen sind deren Durchmesser entsprechend kleiner dimensioniert.

Die Schläuche werden zumindest teilweise, vorzugsweise vollständig, aus transparenten oder transluzenten Materialien gefertigt. Unter transparenten Materialien werden solche verstanden, welche mindestens 80 % des Lichtes im Spektralbereich von 400 nm bis 1000 nm hindurchlassen. Unter transluzenten Materialien werden solche verstanden, welche mindestens 50 % des Lichtes im Spektralbereich von 400 nm bis 1000 nm hindurchlassen. Bevorzugt werden transparente Materialien.

Wesentlich ist, dass diejenigen Bereiche des Schlauches, welche zwischen dem Kultivierungsmedium und der/den Lichtquellen zur Beleuchtung des Kultivierungsmediums angeordnet sind, aus transparenten /transluzenten Materialien gefertigt sind. Befindet sich das Kultivierungsmedium im äußeren Schlauch und das Wärmetauschmedium in einem innenliegenden Segment oder Schlauch, welche jeweils vom Kultivierungsmedium umgeben werden, so kann der das Wärmetauschmedium enthaltende Schlauch bzw. das das Wärmetauschmedium enthaltende Schlauch-Segment aus nicht-transparenten bzw. nicht-transluzenten Materialien gefertigt werden.

Geeignete Materialien sind Glas und Kunststoffe, beispielsweise Homo- oder Copolymerisate wie Polymethylmethacrylat (Plexiglas), Polyester wie PET, Polycarbonat, Polyamid, Polystyrol, Polyethylen, Polypropylen, Polyvinylchlorid oder Siliconmaterialien wie Silicone oder Copolymerisate mit Silicon- und Organocopolymer-Abschnitten.

Für die Bestandteile des Schlauches, welche mit dem Kultivierungsmedium in Berührung kommen, werden Siliconmaterialiem wie Silicone oder Copolymerisate mit Silicon- und Organocopolymer-Abschnitten bevorzugt. Es kann auch so vorgegangen werden, dass die Bestandteile des Schlauches, welche mit dem Kultivierungsmedium in Berührung kommen, mit Siliconmaterialien wie Siliconen oder Copolymerisaten mit Silicon- und Organocopolymer-Abschnitten beschichtet werden, sofern sie nicht aus diesen Materialien gefertigt sind.

Besonders bevorzugt werden die Schläuche aus transparenten oder transluzenten Siliconmaterialien gefertigt.

Zur Herstellung der Schläuche bevorzugte Silicone sind additionsvernetzende Silicone (Siliconkautschuke), wobei die Additionsvernetzung thermisch oder mittels Strahlung eingeleitet werden kann, sowie Copolymerisate mit Silicon- und Organopolymer-Abschnitten (Siliconhybridpolymere).

Additionsvernetzende Siliconkautschuk-Systeme enthalten
a) Organosiliciumverbindungen, die Reste mit aliphatischen Kohlenstoff-Kohlenstoff-Mehrfachbindungen aufweisen,
b) gegebenenfalls Organosiliciumverbindungen mit Si-gebundenen Wasserstoffatomen oder anstelle von a) und b)
c) Organosiliciumverbindungen, die Reste mit aliphatischen Kohlenstoff-Kohlenstoff-Mehrfachbindungen und Si-gebundene Wasserstoffatome aufweisen,
d) die Anlagerung von Si-gebundenen Wasserstoff an aliphatische Mehrfachbindung fördernde Katalysatoren und
e) gegebenenfalls die Anlagerung von Si-gebundenem Wasserstoff an aliphatische Mehrfachbindung bei Raumtemperatur verzögernde Mittel.

Besonders bevorzugte durch Additionsreaktion vernetzende Silicontautschuke sind bei Temperaturerhöhung vernetzende Festsiliconkautschuke (HTV).

Additionsvernetzte HTV-Siliconkautschuke erhält man durch die Vernetzung von mehrfach mit ethylenisch ungesättigten Gruppen, vorzugsweise Vinylgruppen, substituierten Organopolysiloxanen mit mehrfach mit Si-H-Gruppen substituierten Organopolysiloxanen in Gegenwart von Platinkatalysatoren.

Vorzugsweise besteht eine der Komponenten der additionsvernetzenden HTV-2-Siliconkautschuke aus Dialkylpolysiloxanen der Struktur R₃SiO [-SiR₂O]ₙ-SiR₃ mit n ≥ 0, im allgemeinen mit 1 bis 4 C-Atomen im Alkylrest R, wobei die Alkylreste ganz oder teilweise durch Arylreste wie den Phenylrest ersetzt werden können und an einem oder an beiden Enden einer der endständigen Reste R durch eine polymerisierbare Gruppe wie die Vinylgruppe ersetzt ist. Es können aber auch Polymere mit seitenständigen bzw. mit seiten- und endständigen Vinylgruppen verwendet werden. Bevorzugt werden vinylendblockierte Polydimethylsiloxane der Struktur CH₂=CH₂-R₂SiO[-SiR₂O]ₙ-SiR₂-CH₂=CH₂ eingesetzt, sowie vinylendblockierte Polydimethylsiloxane der genannten Struktur, welche noch seitenständige Vinylgruppen tragen. Bei additionsvernetzenden HTV-Siliconkautschuken ist die zweite Komponente ein Copolymer aus Dialkylpolysiloxanen und Polyalkylhydrogensiloxanen mit der allgemeinen Formel R'₃SiO [-SiR₂O]ₙ- [SiHRO]ₘ-SiR'₃ mit m ≥ 0, n ≥ 0 und R in der oben angegebenen Bedeutung und der Maßgabe, dass mindestens zwei SiH-Gruppen enthalten sein müssen, wobei R' die Bedeutung von H oder R haben kann. Es gibt demnach Vernetzer mit seitenständigen und endständigen SiH-Gruppen, während Siloxane mit R'= H, die nur endständige SiH-Gruppen besitzen, auch noch zur Kettenverlängerung verwendet werden. Als Vernetzungskatalysatoren kommen Platinkatalysatoren zum Einsatz. HTV-Siliconkautschuke werden auch als Einkomponentensystem verarbeitet.

Geeignete Materialien sind auch Siliconhybridpolymere. Siliconhybridpolymere sind Copolymerisate oder Pfropfcopolymerisate von Organopolymerblöcken, beispielsweise Polyurethan, Polyharnstoff oder Polyvinylestern, und Siliconblöcken, im allgemeinen auf Basis von Polydialkylsiloxanen der obengenannten Spezifikation. Beispielsweise werden thermoplastische Siliconhybridpolymere in EP 1412416 B1 und EP 1489129 B1 beschrieben, deren diesbezügliche Offenbarung auch Gegenstand dieser Anmeldung sein soll. Derartige Siliconhybridpolymere werden als Thermoplastische Siliconelastomere (TPSE) bezeichnet. Geeignete Materialien sind auch (Kondensations- oder Strahlungs-) vernetzbare Siliconhybridmaterialien, wie in WO 2006/058656 beschrieben,deren diesbezügliche Offenbarung auch Gegenstand dieser Anmeldung sein soll.

Eine detaillierte Übersicht über Silicone, ihre Chemie, Formulierung und Anwendungseigenschaften findet sich beispielsweise in Winnacker/Küchler, "Chemische Technik: Prozesse und Produkte, Band 5: Organische Zwischenverbindungen, Polymere",Seite 1095-1213, Wiley-VCH Weinheim (2005).

Wesentlich für die Hemmung bzw. Verhinderung des Bewuchses mit Mikroorganismen ist die Morphologie der Oberfläche der Siliconschläuche. Die Morphologie der Oberfläche wird über den Kontaktwinkel dieser Oberfläche zu Wasser bestimmt. Bevorzugt sind Oberflächen mit Kontaktwinkeln zwischen 100° und 120°, besonders bevorzugt Oberflächen mit Kontaktwinkeln zwischen 100° und 115°, und ganz besonders bevorzugt Oberflächen mit Kontaktwinkeln zwischen 100° und 113°. Der Kontaktwinkel wird durch die Auswahl der Siliconmaterialien eingestellt. Auf weitere Maßnahmen zur Erhöhung des Kontaktwinkels, beispielweise Aufrauhung der Oberfläche (z.B. Nachahmung des sog. Lotuseffekts), wird vorzugsweise verzichtet. Eine solche Aufrauhung kann nämlich die Kultivierung der phototrophen Mikroorganismen stören. Die Bestimmung des Kontaktwinkels der Oberfläche der Siliconschläuche zu Wasser kann mittels dem Fachmann bekannter Methoden, beispielsweise gemäß DIN 55660-2 erfolgen, unter Einsatz von im Handel erhältlicher Messgeräte zur Bestimmung des Kontaktwinkels, beispielsweise den bei der Fa. Krüss erhältlichen Kontaktwinkel-Messsystemen.

Gegebenenfalls können die genannten additionsvernetzten Silicone übliche Additive zur Haftvermittlung oder übliche Füllstoffe oder Fasermaterialien zur Verbesserung der Mechanik enthalten. Diese Additive werden vorzugsweise maximal in solchen Mengen eingesetzt, dass das Siliconformteil transparent bzw. transluzent bleibt. Es können auch lichtleitende Additive und lichtwellenverschiebende Additive zugegeben werden.

Bevorzugt werden Siliconmaterialien auch zur Beschichtung, der mit dem Kultivierungsmedium in Kontakt tretenden Bestandteile des Schlauches, eingesetzt , insbesondere wenn die Bauelemente nicht aus den genannten Siliconmaterialien gefertigt sind.

Als Beschichtungsmittel bevorzugte Siliconmaterialien sind neben den bereits zur Herstellung der Schläuche genannten Siliconmaterialien noch bei Raumtemperatur durch Kondensation vernetzende Siliconkautschuke, sowie bei Raumtemperatur additionsvernetzende Siliconkautschuke sowie Siliconharze und Silicongele.

Als Beschichtungsmittel geeignete bei Raumtemperatur durch Kondensation vernetzende Siliconkautschuke, sind bei Raumtemperatur vernetzende 1-Komponenten-Systeme, sogenannte RTV-1-Silikonkautschuke. Bei den RTV-1-Siliconkautschuken handelt es sich um Organopolysiloxane mit kondensationsfähigen Endgruppen, welche in Gegenwart von Katalysatoren unter Kondensation bei Raumtemperatur vernetzen. Am gebräuchlichsten sind Dialkylpolysiloxane der Struktur R₃SiO[-SiR₂O]ₙ-SiR₃ mit einer Kettenlänge von n > 2. Die Alkylreste R können gleich oder verschieden sein und haben im allgemeinen 1 bis 4 C-Atome und können gegebenenfalls substituiert sein. Die Alkylreste R können auch teilweise durch andere Reste ersetzt sein, vorzugsweise durch Arylreste, welche gegebenenfalls substituiert sind, und wobei die Alkyl(Aryl)-Gruppen R teilweise durch zur Kondensations-Vernetzung fähige Gruppen ausgetauscht sind, beispielsweise Alkohol- (Alkoxysystem), Acetat- (Essigsäuresystem), Amin- (Aminsystem) oder Oximreste (Oximsystem). Die Vernetzung wird mittels geeigneter Katalysatoren, beispielsweise Zinn- oder Titankatalysatoren katalysiert.

Als Beschichtungsmittel geeignete bei Raumtemperatur durch Kondensation vernetzende Siliconkautschuke, sind auch bei Raumtemperatur vernetzende 2-Komponenten-Systeme, sogenannte RTV-2-Silikonkautschuke. RTV-2-Siliconkautschuke erhält man mittels Kondensationsvernetzung von mehrfach mit Hydroxygruppen substituierten Organopolysiloxanen in Gegenwart von Kieselsäureestern. Als Vernetzer können auch Alkylsilane mit Alkoxy- (Alkoxysystem), Oxim-(Oximsystem), Amin- (Aminsystem) oder Acetatgruppen (Essigsäuresystem) eingesetzt werden, welche in Anwesenheit von geeigneten Kondensations-Katalysatoren, beispielsweise Zinn- oder Titankatalysatoren mit den Hydroxygruppen-terminierten Polydialkylsiloxanen vernetzen.

Beispiele für die in RTV-1 und RTV-2 Siliconkautschuk enthaltenen Polydialkylsiloxane sind solche der Formel (OH)R₂SiO[-SiR₂O]ₙ-SiR₂(OH) mit einer Kettenlänge von n > 2, wobei die Alkylreste R gleich oder verschieden sein können, im allgemeinen 1 bis 4 C-Atome enthalten und gegebenenfalls substituiert sein können. Die Alkylreste R können auch teilweise durch andere Reste ersetzt sein, vorzugsweise durch Arylreste, welche gegebenenfalls substituiert sind. Vorzugsweise enthalten die Polydialkylsiloxane terminale OH-Gruppen, welche mit den Kieselsäureestern bzw. dem System Alkylsilan/Zinn(Titan)katalysator bei Raumtemperatur vernetzen.

Beispiele für die in RTV-1 und RTV-2 Siliconkautschuken enthaltenen, hydrolysierbare Gruppen aufweisende Alkylsilane sind solche der Formel RₐSi(OX)₄₋ₐ, mit a = 1 bis 3 (bevorzugt 1), und X in der Bedeutung von R" (Alkoxysystem), C(O)R" (Essigsäuresystem), N=CR''₂ (Oximsystem) oder NR''₂ (Aminsystem), wobei R" einen einwertigen Kohlenwasserstoffrest mit 1 bis 6 Kohlenstoffatomen bedeutet.

Als Beschichtungsmittel geeignete, bei Raumtemperatur durch Addition vernetzende Siliconkautschuke, sind bei Raumtemperatur vernetzende 1-Komponenten-Systeme, sogenannte additionsvernetzende RTV-1-Siliconkautschuke, bei Raumtemperatur vernetzende 2-Komponenten-Systeme, sogenannte additionsvernetzende RTV-2-Silikonkautschuke oder auch bei Raumtemperatur vernetzende Mehrkomponenten-Systeme. Die Vernetzungsreaktion kann dabei kationisch, mittels entsprechender Katalysatoren, oder radikalisch, mittels Peroxiden, oder durch Strahlung, insbesondere UV-Strahlung, oder thermisch initiiert werden.

Additionsvernetzende RTV-2-Siliconkautschuke erhält man durch mit Pt-Katalysatoren katalysierte Vernetzung von mehrfach ethylenisch ungesättigten Gruppen, vorzugsweise Vinylgruppen, substituierten Organopolysiloxanen mit mehrfach mit Si-H-Gruppen substituierten Organopolysiloxanen in Gegenwart von Platinkatalysatoren.

Vorzugsweise besteht eine der Komponenten aus Dialkylpolysiloxanen der Struktur R₃SiO[-SiR₂O]ₙ-SiR₃ mit n ≥ 0, im allgemeinen mit 1 bis 4 C-Atomen im Alkylrest, wobei die Alkylreste ganz oder teilweise durch Arylreste wie den Phenylrest ersetzt werden können, und an einem oder an beiden Enden einer der endständigen Reste R durch eine polymerisierbare Gruppe wie die Vinylgruppe ersetzt ist. Ebenso können teilweise Reste R in der Siloxankette, auch in Kombination mit den Resten R der Endgruppen, durch polymerisierbare Gruppen ersetzt werden. Bevorzugt werden vinylendblockierte Polydimethylsiloxane der Struktur CH₂=CH₂-R₂SiO[-SiR₂O]ₙ-SiR₂-CH₂=CH₂ eingesetzt.

Die zweite Komponente enthält einen Si-H-funktionellen Vernetzer. Die üblicherweise verwendeten Polyalkylhydrogensiloxane sind Copolymere aus Dialkylpolysiloxanen und Polyalkylhydrogensiloxanen mit der allgemeinen Formel R'₃SiO[-SiR₂O]ₙ-[SiHRO]ₘ-SiR'₃ mit m ≥ 0, n ≥ 0 und der Maßgabe, dass mindestens zwei SiH-Gruppen enthalten sein müssen, wobei R' die Bedeutung von H oder R haben kann. Es gibt demnach Vernetzer mit seitenständigen und endständigen SiH-Gruppen, während Siloxane mit R'= H, die nur endständige SiH-Gruppen besitzen, auch noch zur Kettenverlängerung verwendet werden. Als Vernetzungskatalysator sind geringe Mengen einer platinorganischen Verbindung enthalten.

In jüngster Zeit sind zudem noch spezielle Siliconkautschuke im Handel erhältlich, welche über die beschriebene Additionsreaktion vernetzt werden, indem spezielle Platin-Komplexe bzw. Platin-/Inhibitor-Systeme thermisch und/oder photochemisch aktiviert werden und somit die Vernetzungsreaktion katalysieren.

Siliconharze sind ebenfalls geeignete Materialien für die Herstellung der transparenten oder transluzcnten Beschichtung. Im allgemeinen enthalten die Siliconharze Einheiten der allgemeinen Formel R_{b}(RO)_{c}SiO_{(4-b-c)/2}, worin b gleich 0, 1, 2 oder 3 ist, c gleich 0, 1, 2 oder 3 ist, mit der Maßgabe, dass b+c ≤ 3 ist, und R in der oben dafür angegebenen Bedeutung, welche ein hoch vernetztes Organosilicon-Netzwerk aufbauen. Siliconharze können lösemittelfrei, lösemittelhaltig oder als wässrige Systeme zum Einsatz kommen. Darüberhinaus können auch funktionalisierte, z.B. mit Epoxy- oder Amingruppen funktionalisierte Siliconharze eingesetzt werden.

Silicongele sind ebenfalls geeignete Materialien für die Herstellung der transparenten oder transluzenten Beschichtung. Silicongele werden aus zwei gießbaren Komponenten hergestellt, welche bei Raumtemperatur in Gegenwart eines Katalysators vernetzen. Eine der Komponenten besteht im allgemeinen aus Dialkylpolysiloxanen der Struktur R₃SiO[-SiR₂O]ₙ-SiR₃ mit n ≥ 0, im allgemeinen mit 1 bis 4 C-Atomen im Alkylrest, wobei die Alkylreste ganz oder teilweise durch Arylreste wie den Phenylrest ersetzt werden können, und an einem oder an beiden Enden einer der endständigen Reste R durch eine polymerisierbare Gruppe wie die Vinylgruppe ersetzt ist. Ebenso können teilweise Reste R in der Siloxankette, auch in Kombination mit den Resten R der Endgruppen, durch polymerisierbare Gruppen ersetzt werden. Bevorzugt werden vinylendblockierte Polydimethylsiloxane der Struktur CH₂=CH₂-R₂SiO[-SiR₂O]ₙ-SiR₂-CH₂=CH₂ eingesetzt.

Die zweite Komponente enthält einen Si-H-funktionellen Vernetzer. Die üblicherweise verwendeten Polyalkylhydrogensiloxane sind Copolymere aus Dialkylpolysiloxanen und Polyalkylhydrogensiloxanen mit der allgemeinen Formel R'₃SiO[-SiR₂O]ₙ-[SiHRO]ₘ-SiR'₃ mit m ≥ 0, n ≥ 0 und der Maßgabe, dass mindestens zwei SiH-Gruppen enthalten sein müssen, wobei R' die Bedeutung von H oder R haben kann. Es gibt demnach Vernetzer mit seitenständigen und endständigen SiH-Gruppen, während Siloxane mit R'= H, die nur endständige SiH-Gruppen besitzen, auch noch zur Kettenverlängerung verwendet werden. Als Vernetzungskatalysator sind geringe Mengen einer platinorganischen Verbindung enthalten. Durch das Mischen der Komponenten wird die Vernetzungsreaktion ausgelöst und das Gel gebildet. Diese Vernetzungsreaktion kann durch das Einwirken von Wärme und/oder durch elektromagnetische Strahlung, vorzugsweise UV-Strahlung, beschleunigt werden.

Eine detaillierte Übersicht über Silicone, ihre Chemie, Formulierung und Anwendungseigenschaften findet sich beispielsweise in Winnacker/Küchler, "Chemische Technik: Prozesse und Produkte, Band 5: Organische Zwischenverbindungen, Polymere", Seite 1095-1213, Wiley-VCH Weinheim (2005).

In einer bevorzugten Ausführungsform können die Schlauch-Materialien übliche Additive wie Füllstoffe oder Fasermaterialien zur Verbesserung der Mechanik enthalten. Diese Additive werden vorzugsweise maximal in solchen Mengen eingesetzt, dass das Schlauch-Material transparent bzw. transluzent bleibt. Es können auch lichtleitende Additive und lichtwellenverschiebende Additive zugegeben werden.

Die Fertigung kann mit den etablierten Technologien der Kunststoffverarbeitung erfolgen, die zur Herstellung von Formkörpern eingesetzt werden. Insbesondere bei Siliconen mittels Extrusion oder Spritzgießen zur Formung von thermoplastischen Siliconen (Thermoplast-Spritzgießen), elastomeren Siliconen (Elastomer-Spritzgießen) oder duroplastischen Siliconen (Duroplast-Spritzgießen). Es können aber auch Kombiverfahren, wie z.B. Exjection angewandt werden.

Zur Beschichtung werden die Silicone in flüssiger Form, entweder in Reinsubstanz, als Lösung oder in wässeriger Emulsion aufgetragen. Vorzugsweise beträgt die Viskosität der zur Beschichtung aufzutragenden Flüssigkeit von 10 mPas bis 300.000 mPas. Die Auftragung kann mittels der üblichen Techniken erfolgen, vorzugsweise Streichen, Sprühen, Tauchen, Rakeln, Gießen. Besonders bevorzugt ist hierbei das Tauchen und Sprühen. Es können aber zur Beschichtung von Rohren auch noch weitere Verfahren wie z.B. Schwammauftrag, Schleudern, Extrusion bzw. Querkopfextrusion angewandt werden, sowie für ebene Flächen zusätzlich noch Auftragung mittels Walzenbeschichtung, Rollenbeschichtung oder Pflatschen.

Im Allgemeinen beträgt die Dicke der Beschichtung 10 nm bis 1000 µm, vorzugsweise 1 µm bis 100 µm. Gegebenenfalls können die zu beschichtenden Reaktorteile, zur Verbesserung der Haftung der Silicone, vorbehandelt werden, beispielsweise mittels Corona-Behandlung. Gegebenenfalls können die Silicone übliche Additive zur Haftvermittlung oder übliche Füllstoffe zur Verbesserung der Mechanik enthalten. Diese Additive werden vorzugsweise maximal in solchen Mengen eingesetzt, dass die Siliconbeschichtung transparent bzw. transluzent bleibt.

Die Beleuchtung erfolgt im Allgemeinen mittels Sonnenlicht, welches gegebenenfalls durch Kunstlicht (künstliche Lichtquellen) ergänzt werden kann. Bevorzugt werden zur künstlichen Beleuchtung LEDs enthaltende Beleuchtungsmittel eingesetzt. Geeignet sind aber auch andere künstliche Lichtquellen wie beispielsweise Fluoreszenz-Leuchtstofflampen, Neonlampen, Metalldampf-Lampen, Edelgas-Lampen, Halogenlampen, Schwefelplasmalampen. Bei der Beleuchtung mit künstlichen Lichtquellen können die Kultivierungsbedingungen durch den Einsatz von Lichtquellen mit definierten Wellenlängen, definierter Intensität und gegebenenfalls mittels pulsierender Lichtquellen optimiert werden. Einrichtungen zur künstlichen Beleuchtung werden vorzugsweise im Inneren der Kernstruktur installiert, können aber auch zwischen den Schlauchwicklungen installiert werden. Es ist auch denkbar, die künstlichen Lichtquellen, beispielsweise in Form von LED-Ketten, in eine oder mehrere Kammern der Schläuche des Schlauch-Photobioreaktors einzulegen bzw. einzubauen.

Jeweils an den Enden des Schlauches sind die einzelnen Kammern mit einer zentralen Einheit verbunden, welche den Kreislauf des Kultivierungsmediums und den Kreislauf des Wärmetauschmediums schließen. Diese zentrale Einheit kann ebenfalls ein Schlauch mit mindestens zwei Kammern sein, analog dem Schlauch, welcher für die Wicklung um die Kernstruktur eingesetzt wird. Vorzugsweise sind die das Wärmetauschmedium enthaltenden Schlauchabschnitte mit einem vertikalen, im Innern der Kernstruktur oder außerhalb der Kernstruktur angeordneten Zentralrohr verbunden, welches den Kreislauf für das Wärmetauschmedium schließt. Die das Kultivierungsmedium enthaltenden Schlauchabschnitte sind vorzugsweise ebenfalls mit einem vertikalen, im Innern der Kernstruktur oder außerhalb der Kernstruktur angeordneten Zentralrohr verbunden, welches den Kreislauf für das Kultivierungsmedium schließt.

Bevorzugt wird so verfahren, dass in einem Schlauch, der aus einem äußeren Schlauch und einem koaxial verlaufenden inneren Schlauch zusammengesetzt ist, der innere Schlauch mit Kultivierungsmedium befüllt wird und der äußere Schlauch mit Wärmetauschmedium befüllt wird.

Das die phototrophen Organismen enthaltende Kultivierungsmedium wird im Allgemeinen aus einem Vorratsbehälter in die entsprechenden Kammern des oder der Schläuche gefördert. Die Förderung kann mechanisch mittels einer Pumpe erfolgen, wobei der Förderstrom gepulst erfolgt. Im Schlauch kann die Förderung des Kultivierungsmediums auch mittels Airlift, das heißt mittels Luft oder mittels eines Luft/CO₂-Gemisches oder auch Stickstoff als Trägergas, erfolgen, welches simultan die Versorgung des Kultivierungsmediums mit CO₂ sicherstellt. Die Zuführung von CO₂ oder CO₂ haltigen Gasen kann aber auch, separat und gepulst, über ein Mischsystem oder im Vorlauf der Pumpe erfolgen und damit der Einstellung des pH-Wertes im Kultivierungsmedium dienen.

Bei Betrieb mit einem Airlift sind die hydrodynamischen Verhältnisse bei der Dimensionierung des Schlauch-Photobioreaktors zu berücksichtigen. Prinzipiell kann der Airliftbetrieb in zwei unterschiedlichen Ausführungen durchgeführt werden. Die helikal gewickelten Schläuche werden begast und fungieren als "Riser" und das Zentralrohr dient als "Downer". Umgekehrt kann das Zentralrohr begast werden und die helikal gewickelten Schläuche dienen als "Downer". Bei beiden Anordnungen ist am oberen Ende des Schlauch-Photobioreaktors eine Ausgasungsvörrichtung für den aktiven Gasaustausch zu installieren.

Die Zuführung des Kultivierungsmediums kann beliebig erfolgen, seitlich, von oben oder von unten. Vorzugsweise erfolgt die Zuführung des Kultivierungsmediums am unteren Ende des helikal gewickelten Schlauches bzw. der helikal gewickelten Schlauchabschnitte, falls mehrere separate Schläuche aufgewickelt sind. Das Kultivierungsmedium wird aus den Schläuchen in das vertikale Zentralrohr gefördert und bei entsprechender Dichte der Suspension des Kultivierungsmediums in dessen unterem Abschnitt entnommen. Die Abtrennung der kultivierten Organismen erfolgt in einer Separatoreinheit, beispielsweise mittels Zentrifugätion, Filtration oder Sedimentation.

Das Wärmetauschmedium kann am oberen Ende oder am unteren Ende des helikal gewickelten Schlauches bzw. der helikal gewickelten Schlauchabschnitte in die entsprechenden Kammern eingeleitet werden. Die Förderung erfolgt vorzugsweise pneumatisch mittels Pumpe, im Gleichstrom oder im Gegenstrom zum Kultivierungsmedium. Der Kreislauf des Wärmetauschmediums kann gegebenenfalls eine Wärmetauschereinheit zur Regulierung der Temperatur des Wärmetauschmediums beinhalten. Die Temperatur des Wärmetauschmediums hängt im wesentlichen von der Umgebungstemperatur ab und kann entsprechend eingestellt werden. Vorzugsweise wird der Betrieb des Schlauch-Photobioreaktors mit Automatisierungstechnologie organisiert. Dazu zählen die automatisierte Überwachung und Einstellung von spezifischen Prozessparametern wie Strömungsgeschwindigkeiten, Temperatur, Gasaustausch, Flüssigkeitsaustausch, Dichte bzw. Viskosität, Salzgehalt des Kultivierungsmediums, gegebenenfalls Licht bei künstlicher Beleuchtung (Intensität, Wellenlänge, Hell-/Dunkel-Zyklus, zeitliche Anpassung/Wechsel).

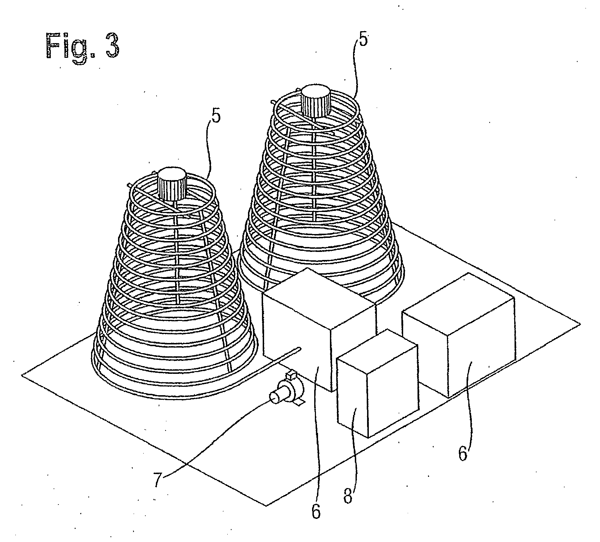

Es können auch mehrere Schlauch-Photobioreaktoren als einzelne Module seriell oder parallel zusammengeschaltet werden. In Figur 3 ist dies grob skizziert wiedergegeben. Zwei Schlauch-Photobioreaktoren 5 sind seriell nebeneinander angeordnet und werden von zwei Vorlagebehältern 6 versorgt. Das Kultivierungsmedium wird über eine gemeinsame Pumpeinheit 7 bewegt. In der Separatoreinheit 8 erfolgt die Abtrennung der phototrophen Organismen vom Kultivierungsmedium.

Der erfindungsgemäße Schlauch-Photobioreaktor hat den Vorteil, dass aufgrund der Kegelstumpf-förmigen Kernstruktur und der helikalen Wicklung des Schlauches der Lichteinfall optimiert und Verschattung reduziert wird. Mittels der Mehrkammerkonstruktion des Schlauches wird die kontinuierliche Temperaturegulation über die gesamte Schlauchlänge gewährleistet, was zum Einen die Kultivierung von Temperaturschwankungs-empfindlicher Mikroorganismen in großem Maßstab ermöglicht, aber auch natürliche, tageszeitbedingte Temperaturschwankungen minimiert. Ein wesentlicher Vorteil beim Einsatz der genannten Siliconmaterialien besteht darin, dass Wandanlagerungen an den Schlauchbereichen, welche mit dem Kultivierungsmedium in Kontakt kommen, stark reduziert werden bzw. gegebenenfalls angelagerte Organismen wesentlich leichter entfernt werden können als bei den üblicherweise eingesetzten Materialien wie Glas.

## Patentansprüche

1. Schlauch-Photobioreaktor mit einer Kegelstumpf-förmigen Kernstruktur und einem oder mehreren transparenten oder transluzenten Schläuchen, welche um die Außenseite und/oder Innenseite der Kernstruktur helikal aufgewickelt sind, **dadurch gekennzeichnet, dass** der transparente oder transluzente Schlauch mindestens zwei Kammern aufweist, von denen mindestens eine von dem Kultivierungsmedium durchströmt wird und mindestens eine von einem Wärmetauschmedium durchströmt wird, **und die Wickelungen des Schlauches in Abstand zueinander gelegt werden, und die Förderung des Kultivierungsmediums gepulst erfolgt.**

2. Schlauch-Photobioreaktor nach Anspruch 1, **dadurch gekennzeichnet, dass** die kegelstumpf-förmige Kernstruktur eine dreidimensionale Struktur ist, mit runder oder polygonaler Grundfläche und im Anstellwinkel α nach innen geneigten Seiten.

3. Schlauch-Photobioreaktor nach Anspruch 1 oder 2, **dadurch gekennzeichnet, dass** der transparente oder transluzente Schlauch aus einem äußeren Schlauch und einem koaxial verlaufenden inneren Schlauch zusammengesetzt ist.

4. Schlauch-Photobioreaktor nach Anspruch 1 bis 3, **dadurch gekennzeichnet, dass** die transparenten oder transluzenten Schläuche aus Glas oder Kunststoff gefertigt sind.

5. Schlauch-Photobioreaktor nach Anspruch 1 bis 4, **dadurch gekennzeichnet, dass** die transparenten oder transluzenten Schläuche aus Siliconmaterialien gefertigt sind.

6. Schlauch-Photobioreaktor nach Anspruch 1 bis 4, **dadurch gekennzeichnet, dass** die transparenten oder transluzenten Schläuche mit Siliconmaterialien beschichtet sind, sofern sie nicht aus diesen Materialien gefertigt sind.

7. Schlauch-Photobioreaktor nach Anspruch 1 bis 6, **dadurch gekennzeichnet, dass** mehrere Schlauch-Photobioreaktoren als einzelne Module seriell oder parallel zusammengeschaltet sind.

8. Verfahren zur Herstellung von phototrophen Organismen mit den Schlauch-Photobioreaktoren gemäß Anspruch 1 bis 7.

9. Verfahren zur Herstellung gemäß Anspruch 8, **dadurch gekennzeichnet, dass** in einem Schlauch, der aus einem äußeren Schlauch und einem koaxial verlaufenden inneren Schlauch zusammengesetzt ist, der innere Schlauch mit Kultivierungsmedium befüllt wird und der äußere Schlauch mit Wärmetauschmedium befüllt wird.

10. Verfahren zur Herstellung gemäß Anspruch 8, **dadurch gekennzeichnet, dass** in einem Schlauch, der aus einem äußeren Schlauch und einem koaxial verlaufenden inneren Schlauch zusammengesetzt ist, der innere Schlauch mit Wärmetauschmedium befüllt wird und der äußere Schlauch mit Kultivierungsmedium befüllt wird, wobei gegebenenfalls der das Wärmetauschmedium enthaltende Schlauch aus nicht-transparenten oder nicht-transluzenten Materialien gefertigt ist.

## Claims

1. Tubular photobioreactor with a core structure of truncated cone shape and one or more transparent or translucent hoses, which are wound helically round the outside and/or inside of the core structure, **characterized in that** the transparent or translucent hose has at least two chambers, with a culture medium flowing through at least one of them and the heat-transfer medium flowing through at least one of them, and the windings of the hose are spaced apart from one another, and the delivery of the culture medium takes place in a pulsed manner.

2. Tubular photobioreactor according to Claim 1, **characterized in that** the core structure of truncated cone shape is a three-dimensional structure, with a rounded or polygonal base and with sides sloping inwards at a tilt angle α.

3. Tubular photobioreactor according to Claim 1 or 2, **characterized in that** the transparent or translucent hose is composed of an outer hose and a coaxially arranged inner hose.

4. Tubular photobioreactor according to Claims 1 to 3, **characterized in that** the transparent or translucent hoses are made of glass or plastic.

5. Tubular photobioreactor according to Claims 1 to 4, **characterized in that** the transparent or translucent hoses are made of silicone materials.

6. Tubular photobioreactor according to Claims 1 to 4, **characterized in that** the transparent or translucent hoses are coated with silicone materials, if they are not made of said materials.

7. Tubular photobioreactor according to Claims 1 to 6, **characterized in that** several tubular photobioreactors are connected together as individual modules in series or parallel.

8. Method of production of phototrophic organisms with the tubular photobioreactors according to Claims 1 to 7.

9. Method of production according to Claim 8, **characterized in that**, in a hose that is composed of an outer hose and a coaxially arranged inner hose, the inner hose is filled with culture medium and the outer hose is filled with heat-transfer medium.

10. Method of production according to Claim 8, **characterized in that**, in a hose that is composed of an outer hose and a coaxially arranged inner hose, the inner hose is filled with heat-transfer medium and the outer hose is filled with culture medium, wherein optionally the hose containing the heat-transfer medium is made of nontransparent or nontranslucent materials.

## Revendications

1. Photo-bioréacteur tubulaire présentant une structure de noyau de forme tronconique et un ou plusieurs tuyau(x) transparent(s) ou translucide(s), qui est/sont enroulé(s) en hélice autour de la face latérale extérieure et/ou de la face latérale intérieure de la structure de noyau, **caractérisé en ce que** le tuyau transparent ou translucide présente au moins deux chambres, dont au moins une est parcourue par le milieu de culture et au moins une est parcourue par un milieu d'échange de chaleur, et les spires du tuyau sont posées à distance les unes des autres, et le transport du milieu de culture est effectué de façon pulsée.

2. Photo-bioréacteur tubulaire selon la revendication 1, **caractérisé en ce que** la structure de noyau de forme tronconique est une structure tridimensionnelle, avec une base circulaire ou polygonale et des faces latérales inclinées vers l'intérieur d'un angle d'inclinaison α.

3. Photo-bioréacteur tubulaire selon la revendication 1 ou 2, **caractérisé en ce que** le tuyau transparent ou translucide est composé d'un tuyau extérieur et d'un tuyau intérieur en position coaxiale.

4. Photo-bioréacteur tubulaire selon les revendications 1 à 3, **caractérisé en ce que** les tuyaux transparents ou translucides sont fabriqués en verre ou en matière synthétique.

5. Photo-bioréacteur tubulaire selon les revendications 1 à 4, **caractérisé en ce que** les tuyaux transparents ou translucides sont fabriqués en matériaux de silicone.

6. Photo-bioréacteur tubulaire selon les revendications 1 à 4, **caractérisé en ce que** les tuyaux transparents ou translucides sont revêtus de matériaux de silicone, dans la mesure où ils ne sont pas fabriqués en ces matériaux.

7. Photo-bioréacteur tubulaire selon les revendications 1 à 6, **caractérisé en ce que** plusieurs photo-bioréacteurs tubulaires sont réunis en série ou en parallèle en modules individuels.

8. Procédé de fabrication d'organismes phototrophes avec les photo-bioréacteurs tubulaires selon les revendications 1 à 7.

9. Procédé de fabrication selon la revendication 8, **caractérisé en ce que**, dans un tuyau qui est composé d'un tuyau extérieur et d'un tuyau intérieur en position coaxiale, on remplit le tuyau intérieur avec un milieu de culture et on remplit le tuyau extérieur avec un milieu d'échange de chaleur.

10. Procédé de fabrication selon la revendication 8, **caractérisé en ce que**, dans un tuyau qui est composé d'un tuyau extérieur et d'un tuyau intérieur en position coaxiale, on remplit le tuyau intérieur avec un milieu d'échange de chaleur et on remplit le tuyau extérieur avec un milieu de culture, dans lequel le tuyau contenant le milieu d'échange de chaleur est éventuellement fabriqué en matériaux non transparents ou non translucides.
